# EUROPEAN PATENT APPLICATION

(11) **EP 3 235 478 A1**
(43) Date of publication of application: **25.10.2017**
(21) Application number: 16165789.5
(22) Date of filing: 18.04.2016
(51) Int. Cl.: A61F 9/02, H04N 13/04

(54) **PROTECTIVE EYEWEAR**

(71) Applicant: Universität Wien, 1010 Wien (AT)
(72) Inventor: GEYER, Philipp, 1100 Vienna (AT); ARNDT, Markus, 1090 Vienna (AT); SEZER, Ugur, 1150 Vienna (AT)
(74) Representative: Sonn & Partner Patentanwälte

(57) **Abstract**

1. Protective eyewear (1) for protection of an operator's eyes (5) against radiation, in particular laser radiation, comprising:
- a head-mounted support (2);
- a camera (12) for generating a video signal of a surrounding of the operator;
- a processing unit (27) for processing the video signal received from the camera (12);
- a display unit (9) for displaying the video signal, the display unit (9) being adapted to be arranged in front of the operator's eyes (5), wherein
- the video camera (12) is a light-field camera.

## Description

The invention relates to protective eyewear for protection of an operator's eyes against radiation, in particular laser radiation.

It is well known that high power lasers used in laboratory environments are capable of inflicting serious damage to an operator's eyes. For this reason, operators are required to wear safety glasses for protection against the hazardous effects of laser radiation.

In the prior art, conventional laser safety glasses are made of materials capable of reflecting or absorbing at the wavelength of the laser light but transmitting other wavelengths. This enables the operator to navigate in the laboratory and perform work. However, such selectively filtering safety glasses entail numerous drawbacks. The known safety glasses have to be designed for absorption at a particular wavelength or wavelength band so that their use is restricted to specific laser equipment. Also, the optical density at the laser wavelength must be sufficiently high to achieve the required safety level. If they block the light entirely, the operator cannot see the lab environment anymore.

Modern lasers have become a serious challenge for protective eye wear, which is illustrated just with a few examples: Firstly, lasers with powers on the Watt level have to be shielded without penetrating through the eye wear to the eye. Current goggles, based on reflecting and absorbing glasses are usually specified for several seconds exposure time, only. Secondly, many lasers emit in a wavelength spectrum that cannot even in principle be entirely shielded by passive eyewear as this would entail blocking out all visible light and leaving the operator in the dark: this holds for supercontinuum lasers, which emit essentially over the entire white light spectrum with an integrated power up to several Watts. This also holds for attosecond lasers, which are Fourier limited to emit in broad spectral bands. Many multifrequency Q-switched lasers emit radiation on individual but widely separated lines, such as for example Nd:YAG lasers, with lines at 1064 nm, 532 nm, 355 nm, 266 nm, 213 nm and pulse energies up to several Joules in the infrared spectrum. Eye protection against all these wavelengths usually requires enormous protective levels, effectively impairing the clear sight on the experiment. This finally also holds for Q-switched or continuous OPO's (optical parametric oscillators) which during a single color scan in spectroscopy applications (over a few seconds) may vary their wavelength continuously for instance from about 200 nm to well beyond 2000 nm, i.e. covering most of all ultraviolet, visible and near infrared wavelengths.

Against this background, a new design of protective eye wear was proposed which replaces the direct eye sight by an entirely opaque screen augmented by a head-mounted camera aimed at the surrounding area of the operator. A video stream from the camera is then displayed on a screen mounted in front of the operator's eyes, behind the protective screen.

For example, such enhanced protective eye wear are disclosed in US 2009/231417 A1 and US 8,334,899.

In this prior art, the camera includes a CCD or CMOS cell and a focusing optics as known from conventional digital cameras. An image reproduction screen is provided in front of the operator's eyes. The output signal from the camera is received by an image processing unit, which drives the image reproduction screen. In this way, the drawbacks of conventional safety glasses with selective absorption at the radiation wavelength are alleviated. The operator is protected against exposure to dangerous light levels which, in the worst case, will only damage an easily replaceable camera while the operator's eyes are shielded behind the screen displaying the video received from the camera.

However, practical experience with known protective goggles having a head-mounted display and a camera for generating a live stream of the surrounding have proven to entail their own deficiencies. In particular, the autofocus of the camera must constantly be adapted to the movements of the operator's head. Despite great improvements in autofocus technology, previously proposed laser protection eyewear ignored the operator's focus of attention in the visual field. This and the adjustment of the optical system produces lags that impair the work of the operator. This is why some operators may experience motion sickness. The present invention aims at improving on known protective eyewear, in particular for protection against high-intensity multicolor laser radiation. It is thus an object of the invention to provide protective goggles that avoid the causes of motion sickness and provide the operator with a working experience as close to direct observation of the environment as possible.

This object is achieved with protective eyewear (goggles) according to claim 1. Preferred embodiments of the invention are contained in the dependent claims.

Thus, the protective goggles of the instant invention at least comprise
- a head-mounted support;
- at least one camera for generating a video signal of a surrounding of the operator;
- at least one processing unit for processing the video signal received from the camera;
- at least one display unit for displaying the video signal, the display unit being adapted to be arranged in front of the operator's eyes, wherein
- the video camera is a light-field camera.

For the purposes of this disclosure, a light field camera, also known as plenoptic camera, is meant to encompass any camera capable of capturing the four-dimensional spatio-angular radiance distribution of the light rays incident on its image sensor. Thus, a light field camera not only captures information about the intensity of light in a scene, but also captures information about the direction that the light rays are traveling in space. In a preferred embodiment, the light field camera is of a type known in the prior art using an array of micro-lenses placed in front of an image sensor to sense intensity, color, and directional information of the incident light. Similar information can be obtained with an array of fixed focus micro-cameras. The depth of field of images captured with the light field camera and making up the video signal is superior to conventional digital cameras. Thus, the light field camera allows larger regions of an image, or even the entire image, to be in focus even for scenes with features arranged over a large depth as usually experienced in a laser laboratory (several centimeters to beyond several meters). Furthermore, the light field camera offers great flexibility for data processing. For example, the processing unit may be arranged for refocusing the images of the video signal after they have been captured. Furthermore, the light field camera can capture a video signal from different perspectives with fewer lags than conventional digital cameras, as there is less need to refocus a lens system during movement of the operator or changes in the surrounding of the operator. The light field camera is particularly advantageous in use with the protective goggles. The increased depth of field is a crucial safety feature as it allows to see sharp on all levels relevant for the operator's work. Enhanced data processing possibilities allow to process and transmit the video signal from the light field camera to the display unit, which in principle is faster than a mechanical lens focusing system. This reduces the occurrence of motion sickness and supports maintaining fine motor skills even with protective eye wear in place. It is an achievement of the invention that the operator may be provided with a clear and bright 3D (full color) vision and sharp focus on all objects in the operator's field of view or in his pupil's line of sight. Because of the possibility of digital processing of the 3D image, focusing corrections can be computed into the image to compensate for the operator's visual defects, if a light-field display is incorporated. This allows for more compact goggles than needed without such correction.

With regard to conventional safety glasses, the camera based protective eyewear inherently provides higher laser safety levels even for stray light. While the eye would be in danger of collimated millimeter sized beams in excess of 1 mW, a camera might sustain higher powers without damage and even provide clear vision at lower light levels.

In a particularly preferable embodiment, the display unit comprises a light field display. Such light field displays have been proposed in the prior art. One example thereof is disclosed in WO 2014186625 A1. Advantageously, the light field display is capable of reproducing the depth features of the images captured with the light field camera. In this way, essentially the entire video stream presented to the operator at the display unit is sharp. Advantageously, the sharpness of the video stream is maintained essentially without lags in case of movements of the operator's head or eyes. A key characteristic of light field displays is their capability to reproduce naturally the depth features of an image. This way the stereoscopic perception and the depth information delivered to each eye can be made consistent. This shall eliminate motion sickness.

In another preferred embodiment, the protective eyewear comprises a display other than a light-field display, for example based on LCD, LED or OLED. The processing unit may be arranged for processing the data from the light field camera such that it creates an overall sharp image. Due to the human eyes' property of seeing sharp only at the point of gaze this embodiment also provides for a natural viewing experience.

In yet another preferred embodiment, the protective eyewear comprises an eye tracker for measuring the point of gaze of the operator. The eye tracker may comprise an eye tracking camera for measuring the direction where the operator is looking, i.e. the point of gaze. Such eye trackers are per se known in the art. This embodiment is particularly advantageous if a light field display is not available. In this case, the display unit may be based on LCD, LED, OLED or other known technology in the prior art. The eye tracker is connected to the processing unit, which, depending on the signal of the eye tracker, processes the data from the light field camera such that the focus of the images displayed on the display unit fits to the point of gaze of the operator. The processing of the signal based on the input from the eye tracker preferably includes an adaptation of the focus. Advantageously, the digital processing of the image data from the light field camera is significantly faster than adaptations of the autofocus in conventional digital cameras, and requires less computing resources than the generation of an overall sharp image.

For shielding the operator's eyes against harmful radiation a preferred embodiment provides for a beam blocker, preferably made of an opaque material, in particular metal or (carbon-)fiber-reinforced plastic, being arranged on the head-mounted support at the back side of the display unit, e.g. between the display unit and the camera. In the normal position of use on the operator's head, the beam blocker is arranged such that harmful radiation, in particular laser radiation, is prevented from reaching the operator's eyes.

In another preferred embodiment, the protective eyewear comprises a back-up camera with a beam blocking device movable between a closed state and an open state. Such mechanical beam blocking device is made from an opaque material for shielding the sensor of the back-up camera in the closed state of the beam blocking device. In the open state of the beam blocking device, the back-up camera may transmit a video stream of the surrounding of the operator to the display unit. Preferably, the processing unit is connected to a drive unit for transferring the beam blocking device of the back-up camera from the closed state to the open state upon detection of a failure of the (primary) camera(s). In an alternative embodiment, the mechanical beam blocker of the back-up camera may be manually moved from the closed state to the open state.

In another preferred embodiment, the protective eyewear comprises a gyro sensor and/or a tilt sensor and/or an accelerometer for monitoring a position of the operator's head. In this embodiment, at least one of the gyro sensor, tilt sensor and accelerometer is connected to the processing unit, in particular for detecting an emergency situation based on the information from the gyro sensor, tilt sensor or accelerometer.

In another preferred embodiment, the protective eyewear comprises a wireless network unit for connecting to a radiation interlock of a radiation source producing the radiation. In this way, a remote control of the radiation source, which preferably is a laser arrangement, may be achieved.

In another preferred embodiment, the processing unit is arranged for activating a radiation interlock for disabling the radiation upon detection of a failure of the camera.

In another preferred embodiment, the protective eyewear comprises an operating unit operable by the operator, the operating unit being arranged for at least one of activating or deactivating the radiation interlock and controlling at least one operating parameter of a radiation source.

By way of example, the invention is further explained with respect to a preferred embodiment shown in the drawings.
Fig. 1 shows a schematic view of a protective eyewear according to an embodiment the invention;
Fig. 2 shows a schematic view of protective eyewear according to another embodiment the invention;
Fig. 3 shows a block diagram of the electronic components of the protective eyewear according to another embodiment of the invention.

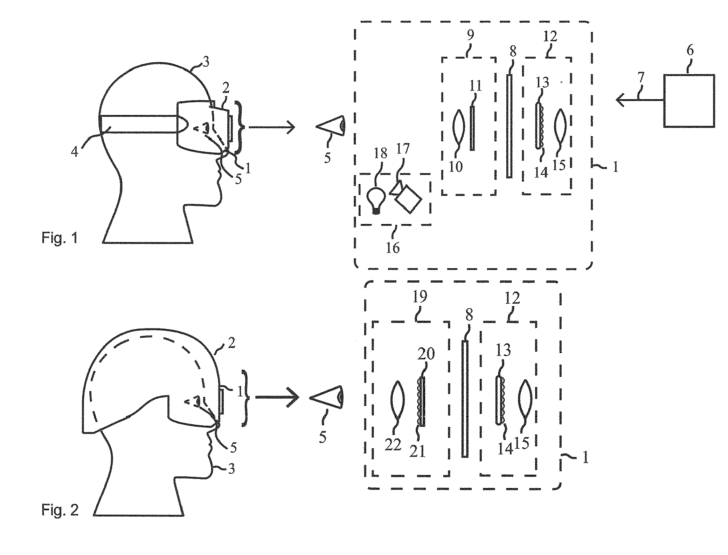

Fig. 1 shows a protective eyewear (or protective goggles) 1 with a head-mounted support 2 to be worn on the head 3 of an operator. According to the embodiment of Fig. 1, the head-mounted support 2 may comprise an optional tightening band 4. In the embodiment of Fig. 2, the head-mounted support 2 takes the shape of a helmet. However, the shape of the protective eyewear 1 is not pertinent for the understanding of the invention. The protective eyewear 1 provides protection of the operator's eyes 5 against the harms otherwise caused by intensive radiation 7 from a radiation source 6 (schematically shown in Fig. 1).

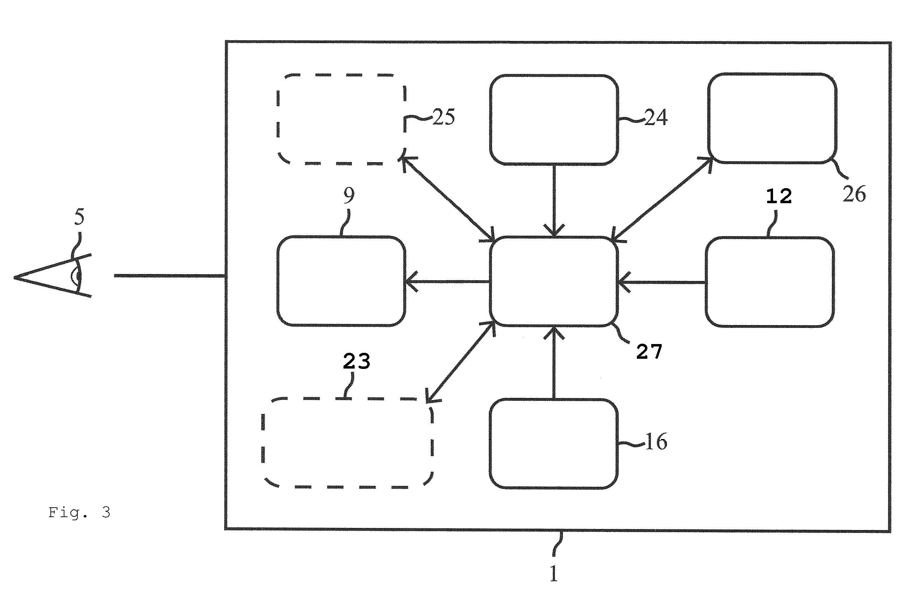

As can be seen in Fig. 1, the protection of the operator's eyes 5 is achieved with a beam blocker 8 at the head-mounted support 2. Facing the operator's eyes 5 and behind the beam blocker 8 a display unit 9 is placed. This display unit 9 contains an optical system 10 arranged in front of or attached to a display 11. The optical system 10 may contain (de-)focusing as well as image correction elements. The display unit 9 of the embodiment shown in Fig. 1 is based on technologies such as LCD, LED, OLED or others. Furthermore, the protective goggles 1 comprise a camera 12 for generating a video stream showing the operator's environment in the laboratory. A processing unit 27 (see Fig. 3) communicates with the camera 12 and the display unit 9, respectively.

It will be understood that the protective goggles 1 preferably comprise two cameras 12 and two display units 9, one for each eye 5 of the operator. Furthermore, a security unit 26 (see Fig. 3) may be present for the case that one of cameras 12 is directly hit by the laser beam 7.

According to the invention, camera 12 is a light field camera having an image capturing sensor 13 with an array of micro-lenses 14. The image capturing sensor 13 is placed behind an optical system 15, which projects the observed image onto the image sensor 13. The image capturing sensor 13 may arise from existing technologies like CCD, CMOS, etc.

As will be explained in greater detail below, the images captured with the light field camera 12 can be processed either to set the sharpness plane after the image was obtained to any position based on eye tracking or an image can be generated which is sharp in its entire extension.

In the embodiment of Fig. 1, for avoiding motion sickness of the operator the images captured with light field camera 12 are processed in processing unit 27 to be overall sharp, or alternatively only focused in these regions where the operator is looking at. In the embodiment of Fig. 1, an optional eye tracker 16 is used to measure the point of gaze of the operator. The eye tracker 16 is connected to the processing (or control) unit 27, which adjusts the focus in the previously captured images such that the images are sharp in direction of the point of gaze of the operator. The eye tracker 16 comprises an eye tracker camera 17 and a light source 18, for example a weak infrared light source, to illuminate the operator's pupil.

In the embodiment of Fig. 2, the display unit 9 comprises a light field display 19 for presenting the images from the light field camera 12 to the operator. The light field display 19 comprises a display 20 with an array of micro-lenses 21 and an appropriate optical system 22. The other components of the protective goggles 1 shown in Fig. 2 may be those of Fig. 1. By using not only a light field camera 12 but also a light field display 19, less processing of the images from the light field camera 12 is required as the light field display 19 is capable of reproducing the depth features of the images. This approach eliminates the motion sickness some users of VR (Virtual Reality) experience, when the image depth features do not match the stereoscopic properties. As a result, the video presented to the operator provides a very natural viewing experience and removes the need of manual focus adjustment and/or having to work with a fixed focus.

Fig. 3 shows a block diagram of the protective goggles 1 as depicted in Fig. 1 but having additional features. As in Fig. 1 and 2, light-field camera 12 sends real time images of the environment of the operator to the processing unit 27 which contains the required computational power for processing the input signal from the light field camera 12 and for outputting processed image data to the display unit 9 or 19. In case of a conventional display unit 9, based on LCD, LED, OLED or the like, the processing unit 27 uses the output signal of an optional eye tracker 16 for focusing the processed images to the operator's point of gaze. However, the optional eye tracker 16 may be dispensed with if a light field display 19 is used, as depicted in Fig. 2, or if an overall sharp image is computed.

As can be seen from Fig. 3, the protective goggles 1 may further comprise an operating unit 24 for manipulation by the operator. The operating unit 24 may comprise a set of operation elements, for example a touchpad or knobs, for regulating operating parameters, such as the brightness of the display unit 9, the wavelengths of the radiation source 6 etc.

Security unit 26, shown in Fig. 3, may comprise at least one backup camera which is mechanically shuttered and is transferred to an open state only when the primary cameras 12 fail due to direct exposure to radiation exceeding the damage threshold of the used cameras 12.

Furthermore, the protective goggles 1 can be augmented with an optional sensor unit 25 comprising a manifold of additional (security) features tracked and triggered by additional sensors. The optionality of these features is highlighted with dotted lines in Fig. 3. Furthermore, sensor unit 25 may comprise a gyro and/or acceleration sensor for monitoring the position of the operator's head 3. The output signal of these sensors may be used for detecting an emergency situation. Other safety features, for example a heart rate monitor or a microphone for communication or recording of audio messages can also be implemented in sensor unit 25.

As can be further seen from Fig. 3, protective goggles 1 may be equipped with a wireless network unit 23, for example a WLAN controller, for connecting to a wireless network. Wireless network unit 23 may be connected via the wireless network to a laser interlock, allowing the wearer of the protective goggles 1 to disable the radiation source 6 in the working place. This may be done through a control panel on the head-mounted support 2. On the other hand, the radiation source 6 can be arranged for automatically disabling the laser interlock when the system notices a failure, for example a failure of camera 12. The control panel may also indicate the on/off status of the radiation source 6 such that the operator knows when it is safe to remove the protective goggles 1 as well as display the status on the lab entering.

## Claims

1. Protective eyewear (1) for protection of an operator's eyes (5) against radiation, in particular laser radiation, comprising:
- a head-mounted support (2);
- a camera (12) for generating a video signal of a surrounding of the operator;
- a processing unit (27) for processing the video signal received from the camera (12);
- a display unit (9) for displaying the video signal, the display unit (9) being adapted to be arranged in front of the operator's eyes (5),
**characterized in that**
- the video camera (12) is a light-field camera.

2. Protective eyewear (1) according to claim 1, **characterized in that** the display unit (9) comprises a light field display (19).

3. Protective eyewear (1) according to claim 1 or 2, **characterized in that** an eye tracker (16) is provided for measuring a point of gaze of the operator.

4. Protective eyewear (1) according to any of claims 1 to 3, **characterized in that** a beam blocker (8) is arranged on the head mounted display (2) at the back side of the display unit (9).

5. Protective eyewear (1) according to any of claims 1 to 4, **characterized by** a back-up camera with a beam blocking device movable between a closed state and an open state.

6. Protective eyewear (1) according to any of claims 1 to 5, **characterized by** a gyro sensor and/or a tilt sensor and/or an accelerometer for monitoring a position of the operator's head (3).

7. Protective eyewear (1) according to any of claims 1 to 6, **characterized in that** a wireless network unit (23) is provided for connecting to a radiation interlock of a radiation source (6).

8. Protective eyewear (1) according to any of claims 1 to 7, **characterized in that** the processing unit (27) is arranged for activating a radiation interlock for disabling the radiation upon detection of a failure of the camera (12).

9. Protective eyewear (1) according to any of claims 1 to 8, **characterized in that** an operating unit (24) is provided, the operating unit (24) being arranged for at least one of activating or deactivating a radiation interlock and controlling at least one operating parameter of a radiation source (6).
